(19) Europäisches Patentamt
European Patent Office
Office européen des brevets

(11) Publication number: 0 268 868 A2

# (12) EUROPEAN PATENT APPLICATION

(21) Application number: 87115997.6

(22) Date of filing: 30.10.87

(51) Int. Cl.[4]: A61K 37/02

(30) Priority: 07.11.86 CS 8061/86

(43) Date of publication of application:
01.06.88 Bulletin 88/22

(84) Designated Contracting States:
BE CH DE FR GB IT LI LU NL SE

(71) Applicant: SPOFA Spojené Podniky Pro Zdravotnickou Vyrobu
Husinecká 11 a
Prag 3(CS)

(72) Inventor: Kasafirek, Evzen, Dipl.-Ing.
Lomena 27
Praha 6(CS)
Inventor: Plaisner, Vaclav
Ricanova 885/30
Praha 6(CS)
Inventor: Korbova, Libuse
Roztylské n. 538
Praha 4(CS)
Inventor: Kohout, Jiri
Roztylské nam. 538
Praha 4(CS)
Inventor: Cizkova, Jirína
Vitkova 24
Praha 8(CS)
Inventor: Ktejci, Ivan
Kovarovicova 113
Praha 4(CS)
Inventor: Pospisil, Arnost
Mezivrsi 46
Praha 4(CS)
Inventor: Pesak, Milan, RNDr
Poljanovova 3158
Praha 4(CS)
Inventor: Sturc, Antonin, Dipl.- Ing.
Kolarova 648/7
Praha 4(CS)
Inventor: Krepelka, Jiri, Dipl.- Ing.
Madridska 26
Praha 10(CS)
Inventor: Dlabac, Antonin
Jasminova 10
Praha 10(CS)
Inventor: Vanzura, Jiri
Nam.Osvoboditelu 820
Hradec Kralové(CS)

EP 0 268 868 A2

(74) Representative: **Patentanwälte Beetz sen. - Beetz jun. Timpe - Siegfried - Schmitt-Fumian**
**Steinsdorfstrasse 10**
**D-8000 München 22(DE)**

(54) **Pharmaceutical compositions for the treatment of gastro-intestinal diseases.**

(57) The invention relates to pharmaceutical compositions for the treatment of gastro-intestinal diseases, especially gastric and duodenal ulcers, which comprise as a physiologically active ingredient the spirocyclic dipeptide cyclo(L-alanyl-1-amino-1-cyclopentanecarbonyl), preferably together with a physiologically inert carrier substrate, and optionally an adjuvant, physiologically active component. The subject composition is designated for oral or parenteral administration; it has a very low toxicity, is well tolerated and acts beneficially even at low dosage levels.

# Pharmaceutical compositions for the treatment of gastro-intestinal diseases

The invention relates to compositions for the treatment of gastro-intestinal diseases, especially gastric and duodenal ulcers, designated preferably for oral administration.

Gastric and duodenal ulcers pertain to frequent diseases of the gastro-intestinal tract. Heretofore the ulcus therapy involves, beyond appropriate diet regimens, causal and/or palliative medication, predominantly with antacids (acting due to a protective adsorption effect) and anticholinergic agents of the gastric $H_2$ receptor antagonist type; this medication is usually accompanied by certain adverse side-effects, e.g. dryness of the mouth, constipation and anorexia.

It is the object of this invention to provide new pharmaceutical compositions for the treatment of gastro-intestinal diseases, which can be orally adminstered and are not accompanied by the above-mentioned undesired side-effects, wherein a new active ingredient is used.

The above object is achieved according to claim 1.

The pharmaceutical compositions according to the invention for the treatment of gastro-intestinal diseases as a physiologically active ingredient a specific spirocyclic dipeptide, cyclo-(L-alanyl-1-amino-1-cyclopentanecarbonyl) of formula I

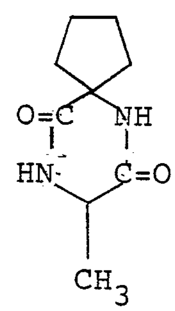

(I),

optionally in combination with a physiologically inert vehicle or carrier substrate and, if required, an adjuvant antacid, topical pain-relieving, spasmolytic and/or sedative component.

Accordingly, the invention relates also to the use of the compound of formula I for formulating pharmaceutical composition for the treatment of gastrointestinal diseases.

The afore-named compound of formula I is already known (CS authors certificate 231 227 and corresponding GB-B 2 127 807, FR-B 83 15599 and CH-B 655 929); it was originally developed and tested in connection with an experimental study of new spirocyclic dipeptides and their effect on the central nervous system.

Now it has been surprisingly found that this compound significantly stimulates the growth of eubiotic, healthy diploid cells from lungs. This remarkable, unexpected observation allowed to assume that compound I has a curative, i.e. regenerating effect on cells and tissues damaged under the action of endogenous factors, e.g. as a result of insufficiently inhibited peptic (proteolytic) action on mucous membranes that elicits formation of gastric and duodenal ulcers. Compound I proved to be substantially non-toxic and free of undesired adverse side-effects. Its beneficial action on said peptic ulcers was confirmed by pharmacological tests in vivo, on experimental ulcus disease models of various nature, e.g. those induced in animals by the ligature of pylorus (method of Shay) or by administration of reserpin, whereas negative results (without pronounced improvement) were obtained on ulcus models after administration of indomethacin or kebuzone (ketophenylbutazone), i.e. 4-(3-oxobutyl)-1,2-diphenylpyrazolidine-3,5-dione; this indicates that compound I has not a distinctly beneficial effect on ulcus diseases induced by improper use of antirheumatic drugs. The experiments were conducted with male Wistar rats of 200 to 250 g body mass.

1. Pylorus ligature model

The rats under test were kept starvine for a period of 24 hours, with water supply ad libitum. Compound I was dissolved in isotonic saline and administered intramuscularly at three dosage levels, viz. in doses of 1 mg, 2 mg or 3 mg per animal, immediately after application of the ligature. After 18 hours the animals were sacrificed and evaluated; the results are summarized in Table 1.

Table 1

| Dosage (mg) | Points[a)] (average) | Gastric juice volume (ml) | HCl (mmol/ml) | Amylase[b)] (mmol/ml) |
|---|---|---|---|---|
| Control | 38 | 14,8 | 0,87 | 0,33 |
| 1 | 45 | 12,6 | 0,67 | 0,31 |
| 2 | 11 | 14,9 | 0,92 | 0,32 |
| 3 | 30 | 16,1 | 1,05 | 0,42 |

Notes: a) ulcus response evaluation system according to Vokác et al., Csl. Gastroenterol. Výz. 11 (1957) 22.

b) determined by the orcinol method, Amer. Rev. Tuberc. 68 (1952) 594.

The data of Table 1 show that an i.m. dose of compound I of 2 mg elicited a decrease in the average ulcus size and count by 72 %; the reported decrease is highly significant (Student t-test p lower than 0,001). On the contrary, no substantial differences in the gastric juice quantity, acidity and enzymatic activity were observed, which suggests probable low incidence of adverse side-effects or digestive dysfunction during therapy.

2. Reserpin model

The experimental ulcus was induced by intramuscular administration of reserpin (10 mg) to rats kept without food for 24 hours. The results are summarized in Table 2.

Table 2

| Dosage (mg) | | Points[a)] (average) | Proteases[b)] | Proteins[c)] | Hexoses[d)] |
|---|---|---|---|---|---|
| Control | C1 | 33 | 0,32 | 25 | 0,45 |
| | C2 | 11 | 0,25 | 27 | 0,46 |
| 2 | | 3,8 | 0,24 | 28 | 0,51 |

Notes: a) Vokác et al., loc-cit. (cf. Table 1, note a)).

b) mmol/g of tissue, J.Gen.Physiol. 16 (1932) 59.

c) mg/g of tissue, Lowry et al., J.Biol.Chem. 193 (1951) 265.

d) mg/g of tissue, Amer.Rev.Tuberc. 68 (1952) 594.

The tabulated data indicate that the same 2 mg i.m. dose of compound I elicited a highly significant decrease in the reserpin ulcus size and count. The observed decrease in proteolytic activity was moderate to slight, changes in proteins and hexoses were clearly unsignificant.

The subject compound of formula I can be administered, in accordance with kind and extent of the treated lesion, either parenterally (e.g. by injections) or orally, by ingestion of any convenient oral dispensing form (tablets, coated tablets, capsules, dragees, suspensions and the like); the administration route, pharmaceutical dosage unit and medical dosage schedule are tailored individually, depending on the nature and severity of the disease and the tolerance of the patient. Usual dosage involves 3 to 6 tablets of 10 mg daily q.d. until disappearance of symptoms or according to X-ray examination findings. For rapid mastering of acute lesions it is advisable, at least during the initial stage of the treatment, to prefer the parenteral route of administration, at an approximate dosage level of three times a day 5 mg doses in the form of e.g. 5 ml injections. After achieving the desired introductory improvement, this parenteral therapy is conveniently replaced by the aforementioned oral treatment; this may be continued, thanks to substantial non-toxicity and very good tolerance of the subject agent, over a prolonged time period, if required for several weeks to several months.

In the following the invention will be explained in connection with the subject composition and prefer able procedures for its formulation with reference to examples.

Example 1 - Injections

| | |
|---|---|
| Compound of formula I | 0,100 g |
| Mannitol | 4,800 g |
| Water for injections | ad 100,0 ml. |

Compound I and mannitol are successively dissolved in water for injections, and the resulting solution is sterilized by filtration and aseptically filled into ampoules of 5 ml volume. The obtained injections, when storaged in cold and dark, are stable for at least one year.

Example 2 - Tablets (direct tableting)Compound of formula I 10,00 g
Calcium hydrogen phosphate dihydrate 154,60 g
Maize starch 32,50 g
Magnesium stearate 1,90 g.

Compound I is successively mixed with maize starch, calcium hydrogen phosphate and magnesium stearate. The obtained powdery mixture is thoroughly homogenized and directly tableted on a rotary tableting machine to give tablet cores of 7 mm diameter and 190 mg mass. The resulting cores are optionally coated with a polymeric film-forming material, e.g. hydroxypropyl methyl cellulose, or a sugar solution.

Example 3 - Tablets (via granulation)Compound of formula I 10,00 g
Microcrystalline cellulose 7,00 g
Lactose 86,80 g
Maize starch 42,00 g
Sodium carboxymethyl starch 2,80 g
Magnesium stearate 1,40 g.

Compound I is successively mixed with lactose and the main portion (36,4 g) of maize starch and homogenized. The homogenizate is granulated with use of the remaining amount (5,6 g) of maize starch in the form of a 12,5 % hydrogel. The so formed wet granulate is dried at a temperature of 50 °C, and the dry material is passed through a sieve (1 × 1 mm sieve mesh). The remaining solids (microcrystalline cellulose, sodium carboxymethyl starch and magnesium stearate) are added, and the mixture is homogenized and tableted on a rotary tableting machine to give tablet cores of 7 mm diameter and 150 mg mass. If required, the core surface is coated as described in example 2.

Example 4 - Tablets (via granulation with polyvinylpyrrolidone)Compound of formula I 10,00 g
Calcium hydrogen phosphate dihydrate 20,00 g
Lactose 102,00 g
Maize starch 20,00 g
Polyvinylpyrrolidone 3,20 g
Sodium carboxymethyl starch 3,20 g
Magnesium stearate 1,60 g.

Compound I is mixed with calcium hydrogen phosphate, lactose and maize starch. The powdery mixture is homogenized, granulated with use of polyvinylpyrrolidone in the form of an 15 % aqueous solution, and the obtained granulate is dried and sieved as described in example 3. The remaining solids (sodium carboxymethyl starch and magnesium stearate) are then added, and the material is processed as above to obtain tablets of 7 mm diameter and 160 mg mass.

Example 5 -Composite tablets (via granulation)Compound of formula I 10,00 g
Oxyphenone hydrobromide (2-ethyl-aminoethyl-α-phenyl-α-cyclohexylglycolate) 2,00 g
Calcium hydrogen phosphate dihydrate 152,60 g
Maize starch 32,50 g
Magnesium stearate 1,90 g.

Compound I is successively mixed with oxyphenone hydrobromide, calcium hydrogenphosphate dihydrate and maize starch; the mixture is granulated as described in example 3; thereafter, magnesium stearate is admixed, and the material is processed as above to obtain tablets of 7 mm diameter and 150 mg mass. Oxyphenone is a commercial spasmolytic and parasympatholytic agent.

Example 6

| | |
|---|---|
| Compound of formula I | 10,00 g |
| Magnesium trisilicate | 100,00 g |
| Colloidal aluminum phosphate | 400,00 g |
| Lactose | 48,00 g |
| Polyvinylpyrrolidone | 12,00 g |
| Sodium carboxymethyl starch | 18,00 g |
| Magnesium stearate | 12,00 g. |

The formulation procedure is similar as described hereinbefore in example 4, with the only exception that tablet cores of 600 mg mass are prepared.

Example 7

| | |
|---|---|
| Compound of formula I | 10,00 g |
| Diazepam (7-chloro-1,3-dihydro-1-methyl-5-phenyl-2H-1,4-benzodiazepin-2-one) | 2,00 g |
| Calcium hydrogen phosphate dihydrate | 152,60 g |
| Maize starch | 32,50 g |
| Magnesium stearate | 1,90 g. |

Compound I is mixed successively with diazepam and the above excipients, and the powdery mixture is processed to obtain tablet cores substantially by the direct tableting procedure of example 2.

## Claims

1. Pharmaceutical compositions for the treatment of gastro-intestinal diseases, particularly gastric und duodenal ulcers, which comprise as a physiologically active ingredient cyclo-(L-alanyl-1-amino-1-cyclopentanecarbonyl) of formula I

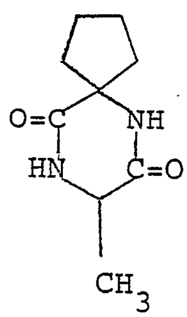

(I),

optionally in combination with a physiologically inert carrier substrate, and, if required, an adjuvant antacid, topically pain-relieving, spasmolytic and/or sedative component.

2. Pharmaceutical compositions according to claim 1 comprising 2-ethylaminoethyl-α-phenyl-α-cyclohexylglycolate as said adjuvant.

3. Use of cyclo-(L-alanyl-1-amino-1-cyclopentanecarbonyl) for the formulation of pharmaceutical compositions for the treatment of gastro-intestinal diseases.